# EUROPEAN PATENT APPLICATION

(11) **EP 3 819 638 A1**
(43) Date of publication of application: **12.05.2021**
(21) Application number: 19846908.2
(22) Date of filing: 19.04.2019
(51) Int. Cl.: G01N 33/53, G01N 21/64, G01N 33/531, G01N 33/543

(54) **METHOD FOR MEASURING BRAIN NATRIURETIC PEPTIDE AND KIT FOR MEASURING BRAIN NATRIURETIC PEPTIDE**

(30) Priority: 06.08.2018 JP 2018147725
(71) Applicant: Konica Minolta, Inc., Tokyo 100-7015 (JP)
(72) Inventor: MURAYAMA, Takanori, Tokyo 100-7015 (JP)
(74) Representative: Gille Hrabal Partnerschaftsgesellschaft mbB Patentanwälte
(86) International application number: PCT/JP2019/016737
(87) International publication number: WO 2020/031436

(57) **Abstract**

The present invention pertains to a BNP measuring method with which it is possible to measure the concentration of BNP in a specimen with higher sensitivity and higher reproducibility. A protease inhibitor is added to a specimen that contains brain natriuretic peptide to obtain a pretreatment specimen. The pretreatment specimen is provided on a metal film to which a binding substance capable of binding specifically. Either prior to or following the binding to the binding substance, the brain natriuretic peptide is labeled with a fluorescent substance. In a state where the brain natriuretic peptide labeled with the fluorescent substance is bound to the binding substance immobilized to the metal film, the metal film is irradiated with excitation light so as to cause surface plasmon resonance to occur in the metal film, and then fluorescence released from the fluorescent substance is detected.

## Description

### Technical Field

The present invention relates to a measurement method for brain natriuretic peptide and a measurement kit for brain natriuretic peptide.

### Background Art

Brain natriuretic peptide (hereinafter also referred to as "BNP") is a hormone consisting of 32 amino acids, and synthesized principally in the ventricle to be secreted into the blood. It is known that a blood concentration of BNP increases in a patient suffering from ventricle dysfunction such as cardiac arrest, cardiac hypertrophy, and myocardial infarction. Therefore, a blood concentration of BNP is clinically significant as an index corresponding to the severity of such ventricle dysfunction.

Various methods have been developed as a method for measuring a blood concentration of BNP. For example, PTL 1 discloses a method for measuring a concentration of BNP by a sandwich immunoassay using a magnetic particle as a solid phase.

### Citation List

### Patent Literatures

PTL 1: Japanese Patent Application Laid-Open No. 2010-032360

### Summary of Invention

### Technical Problem

Although various methods for measuring a blood concentration of BNP have been developed as described above, there is a demand for a method by which a blood concentration of BNP can be measured with higher sensitivity and higher reproducibility.

An object of the present invention is to provide a measurement method for BNP and a measurement kit for BNP by which a concentration of BNP in a specimen can be measured with higher sensitivity and higher reproducibility than in a conventional measurement method.

### Solution to Problem

A measurement method for brain natriuretic peptide, according to one embodiment of the present invention includes: preparing a measurement chip including a metal film and a binding substance which is immobilized on the metal film and which specifically binds to brain natriuretic peptide; obtaining a pretreated specimen by adding, to a specimen containing brain natriuretic peptide, one, two or more protease inhibitors selected from the group consisting of a serine protease inhibitor and a cysteine protease inhibitor; providing the pretreated specimen onto the metal film to cause the brain natriuretic peptide contained in the pretreated specimen to be bound to the binding substance; labeling, with a fluorescent substance, the brain natriuretic peptide before or after binding to the binding substance; and detecting fluorescence emitted from the fluorescent substance when the metal film is irradiated with excitation light in such a manner as to generate surface plasmon resonance in the metal film with the brain natriuretic peptide labeled with the fluorescent substance kept in a state bound to the binding substance.

A measurement kit for brain natriuretic peptide, according to one embodiment of the present invention includes: a measurement chip including a metal film, and a binding substance which is immobilized on the metal film and which specifically binds to brain natriuretic peptide; a pretreatment agent containing one, two or more protease inhibitors selected from the group consisting of a serine protease inhibitor and a cysteine protease inhibitor; and a labeling reagent for labeling brain natriuretic peptide with a fluorescent substance.

### Advantageous Effects of Invention

According to the present invention, a concentration of BNP in a specimen can be measured with higher sensitivity and higher reproducibility than in a conventional measurement method.

### Brief Description of Drawings

FIG. 1 is a flowchart illustrating an example of a measurement method for brain natriuretic peptide according to an embodiment;
FIG. 2A is a schematic cross-sectional view illustrating a structure of a measurement chip for use in PC-SPFS, and FIG. 2B is a schematic cross-sectional view illustrating a structure of a measurement chip for use in GC-SPFS;
FIG. 3 is a schematic cross-sectional view illustrating an example of a measurement chip for use in PC-SPFS;
FIG. 4 is a graph corresponding to the relationship, obtained when aprotinin is used as a protease inhibitor, between an aprotinin concentration and measurement reproducibility (variation coefficient); and
FIG. 5 is a graph corresponding to the relationship, obtained when benzamidine is used as a protease inhibitor, between a benzamidine concentration and measurement reproducibility (variation coefficient).

### Description of Embodiments

Now, embodiments of the present invention will be described in detail with reference to the accompanying drawings.

### [Measurement Method for Brain Natriuretic Peptide]

In a measurement method for brain natriuretic peptide (BNP) of the present embodiment, BNP is measured by utilizing surface plasmon-field enhanced fluorescence spectroscopy (hereinafter also referred to as "SPFS"). In SPFS, a fluorescent substance is excited to emit fluorescence by an electric field enhanced by surface plasmon resonance (hereinafter also referred to as "SPR"), and therefore, as compared with general fluoroimmunoassay, a target (that is, BNP in the present embodiment) can be detected with high sensitivity. In SPFS, whole blood can be used as a specimen.

According to a preliminary experiment made by the present inventors, however, there was a room of improvement in reproducibility of measurement results obtained by the measurement of a concentration of BNP in blood by SPFS. The reason is not clear, but the present inventors presume as follows (which is not restrictive).

In SPFS by which a target can be measured with high sensitivity, merely a small amount of a specimen (such as blood or a diluted solution thereof) is required. In this case, in order to cause a binding substance for BNP (such as an anti-BNP antibody) immobilized in a reaction field to definitely capture a slight amount of BNP contained in a specimen, a mechanical stimulus is preferably applied to the specimen in the reaction field. Thus, a probability that BNP contained in the specimen comes into contact with the binding substance immobilized in the reaction field is increased, and hence measurement accuracy for the BNP can be improved.

Here, if a small amount of blood or a diluted solution thereof is used as a specimen, opportunities for erythrocytes contained in the specimen to come into contact with a surface of an operation tool, a measurement device or the like increase, and hence the erythrocytes contained in the specimen are easily destroyed. Besides, if a mechanical stimulus is applied to the specimen present in the reaction field for improving the measurement accuracy for BNP, the erythrocytes contained in the specimen are more easily destroyed. If the erythrocytes are destroyed for these reasons, protease present in the erythrocytes leaks out, and hence a part of BNP is decomposed by protease.

Since a part of BNP is thus decomposed if the amount of protease in the specimen changes in a very short period of time during the measurement operation, it is presumed that there is a room of improvement in reproducibility of measurement results in highly sensitive SPFS.

The present inventors made earnest studies based on the above-described idea, and as a result, have found that BNP can be measured with high reproducibility by highly sensitive SPFS by adding a protease inhibitor to a specimen at a stage of a pretreatment of the measurement, and thus, a measurement method for BNP according to the present embodiment was accomplished.

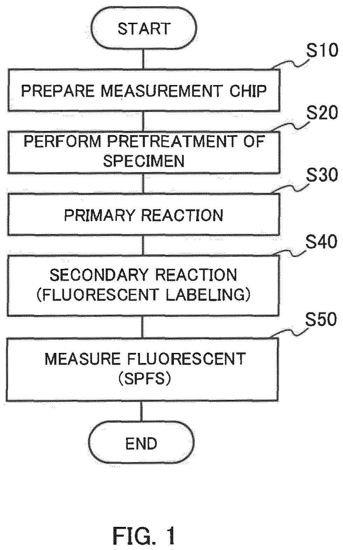

Now, the measurement method for BNP of the present embodiment will be specifically described. FIG. 1 is a flowchart illustrating an example of the measurement method for BNP of the present embodiment.

### (Preparation of Measurement Chip)

First, a measurement chip including a metal film and a binding substance specifically binding to a BNP is prepared (step S10). In SPFS, SPR is generated by causing evanescent waves, caused by irradiating a metal film with light (that is, excitation light in the present embodiment), and surface plasmon to couple to each other. As a method for generating SPR, a method in which a prism is disposed on one plane of a metal film (Kretschmann configuration), a method in which a diffraction grating is formed in a metal film, and the like are known. SPFS employing the former method is designated as prism coupling (PC)-SPFS, and SPFS employing the latter method is designated as grating coupling (GC)-SPFS. The measurement method for a BNP of the present embodiment may employ either of PC-SPFS and GC-SPFS.

As described above, when a metal film is irradiated with excitation light, SPR is generated. The type of a metal constituting the metal film is not particularly limited as long as the metal can generate SPR. Examples of the metal constituting the metal film include gold, silver, copper, aluminum and an alloy thereof.

The binding substance can specifically bind to a BNP, and is immobilized on the metal film for capturing a BNP contained in a specimen. In general, the binding substance is uniformly immobilized in a prescribed region (reaction field) on the metal film. The type of the binding substance immobilized on the metal film is not particularly limited as long as it can specifically bind to a BNP. Examples of the binding substance include an antibody capable of specifically binding to a BNP (an anti-BNP antibody), a nucleic acid capable of specifically binding to a BNP, a lipid capable of specifically binding to a BNP, and a protein, excluding an antibody, capable of specifically binding to a BNP. When the binding substance is an anti-BNP antibody, the anti-BNP antibody may be a monoclonal antibody, a polyclonal antibody, or a fragment of an antibody. One or two or more binding substances may be immobilized on the metal film. For example, the anti-BNP antibody immobilized on the metal film may be one or two or more anti-BNP monoclonal antibodies or anti-BNP polyclonal antibodies.

A method for immobilizing the binding substance is not particularly limited. For example, a self-assembled monolayer (hereinafter referred to as "SAM") or a polymer film to which the binding substance (such as an anti-BNP antibody) is caused to bind may be formed on the metal film. An example of the SAM includes a film made of a substituted aliphatic thiol such as HOOC-(CH₂)₁₁-SH. Examples of a material constituting the polymer film include polyethylene glycol and MPC polymer. A polymer having a reactive group (or a functional group that can be converted into a reactive group) capable of binding to the binding substance (such as an anti-BNP antibody) may be immobilized on the metal film, and the binding substance (such as an anti-BNP antibody) may be caused to bind to the polymer.

A measurement chip is a structure having each side with a length preferably of several mm to several cm, and may be a more compact structure or a larger structure not belonging to the category of "chip".

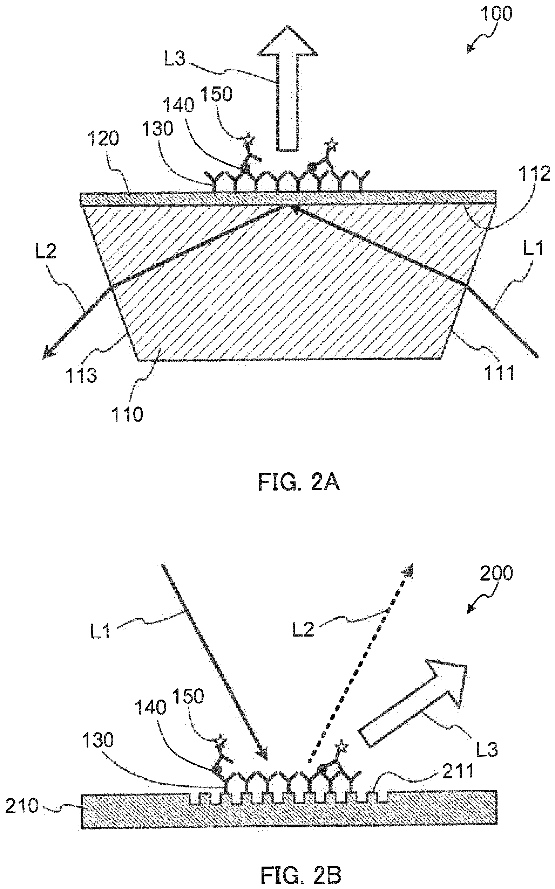

FIG. 2A is a schematic cross-sectional view illustrating the structure of a measurement chip for use in PC-SPFS, and FIG. 2B is a schematic cross-sectional view illustrating the structure of a measurement chip for use in GC-SPFS. For convenience of description, the sizes and the shapes of the respective constituent elements are not accurate in these drawings. These drawings show examples in which an anti-BNP antibody is used as the binding substance.

As illustrated in FIG. 2A, measurement chip 100 for use in PC-SPFS includes prism 110, metal film 120 and (a layer of) anti-BNP antibody 130. Prism 110 is made of a dielectric transparent to excitation light LI, and includes entrance surface 111 where excitation light L1 enters, film surface 112 on which excitation light L1 reflects, and exit surface 113 through which reflected light L2 exits. The shape of prism 110 is not particularly limited. In the exemplified case of FIG. 2A, prism 110 is in a column shape having a trapezoidal bottom. A surface corresponding to one base of the trapezoid is film surface 112, a surface corresponding to one leg is entrance surface 111, and a surface corresponding to the other leg is exit surface 113. Examples of a material of prism 110 include a resin and glass. The material of prism 110 is preferably a resin having a refractive index for excitation light of 1.4 to 1.6 and having low birefringence. Metal film 120 is disposed on film surface 112 of prism 110. The method for forming metal film 120 is not particularly limited. Examples of the method for forming metal film 120 include sputtering, deposition and plating. A thickness of metal film 120 is not particularly limited, and is preferably in a range of 30 to 70 nm.

As illustrated in FIG. 2A, when metal film 120 is irradiated with excitation light L1 through prism 110 so as to generate SPR in metal film 120, an electric field enhanced by SPR is generated in the vicinity of metal film 120. At this point, when BNP 140 labeled with fluorescent substance 150 binds to anti-BNP antibody 130 disposed on metal film 120, fluorescent substance 150 is excited by the enhanced electric field to emit fluorescence L3.

As illustrated in FIG. 2B, measurement chip 200 for use in GC-SPFS includes metal film 210 having diffraction grating 211 formed therein and (a layer of) anti-BNP antibody 130. The method for forming metal film 210 is not particularly limited. Examples of the method for forming metal film 210 include sputtering, deposition and plating. The thickness of metal film 210 is not particularly limited, and is preferably in a range of 30 to 500 nm. The shape of diffraction grating 211 is not particularly limited as long as evanescent waves can be caused. For example, diffraction grating 211 may be a one-dimensional diffraction grating or a two-dimensional diffraction grating. For example, as a one-dimensional diffraction grating, a plurality of convexities parallel to one another are formed at prescribed intervals on the surface of metal film 210. As a two-dimensional diffraction grating, projections in a prescribed shape are periodically arranged on the surface of metal film 210. Examples of arrangement of the projections include square lattice arrangement and triangular (hexagonal) lattice arrangement. Examples of a cross-sectional shape of diffraction grating 211 include a square wave shape, a sine wave shape and a sawtooth shape. A method for forming diffraction grating 211 is not particularly limited. For example, metal film 210 may be provided with a concave/convex shape after being formed on a plate-shaped substrate (not shown). Alternatively, metal film 210 may be formed on a substrate (not shown) precedently provided with a concave/convex shape. No matter which method is employed, metal film 210 including diffraction grating 211 can be formed.

As illustrated in FIG. 2B, when metal film 210 (diffraction grating 211) is irradiated with excitation light L1 so as to generate SPR in metal film 210 (diffraction grating 211), an electric field enhanced by SPR is generated in the vicinity of metal film 210 (diffraction grating 211). At this point, when BNP 140 labeled with fluorescent substance 150 binds to anti-BNP antibody 130 disposed on metal film 210 (diffraction grating 211), fluorescent substance 150 is excited by the enhanced electric field to emit fluorescence L3.

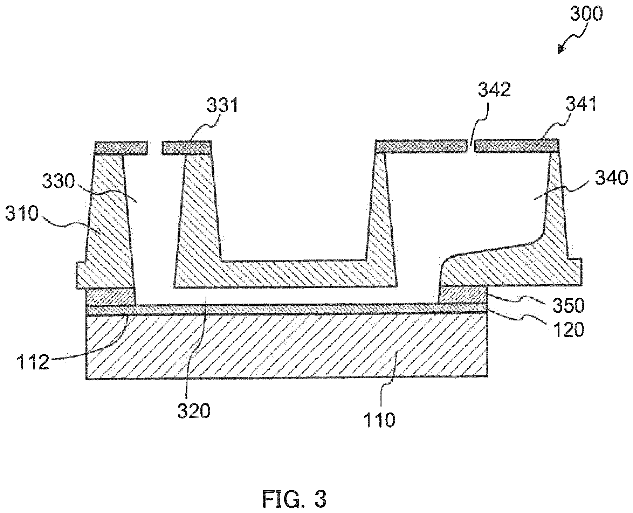

FIG. 3 is a schematic cross-sectional view illustrating an example of the measurement chip for use in PC-SPFS. As illustrated in FIG. 3, measurement chip 300 includes prism 110 having entrance surface 111, film surface 112 and exit surface 113, metal film 120 formed on film surface 112 of prism 110, and passage cover 310 disposed on film surface 112 of prism 110 or metal film 120. In FIG. 3, entrance surface 111 and exit surface 113 are present respectively in front of and behind a sheet surface of the drawing. Measurement chip 300 further includes passage 320, liquid injection port 330 connected to one end of passage 320, and reservoir 340 connected to the other end of passage 320. In the present embodiment, passage cover 310 is caused to adhere to metal film 120 (or prism 110) through adhesive layer 350 of a double sided tape or the like, and adhesive layer 350 also plays a role to define the shape of a side surface of passage 320. Although not illustrated in FIG. 3, anti-BNP antibody 130 is immobilized in a partial region (reaction field) of metal film 120 exposed in passage 320. Liquid injection port 330 is closed by liquid injection port covering film 331, and reservoir 340 is closed by reservoir covering film 341. Reservoir covering film 341 is provided with vent 342.

Passage cover 310 is made of a material transparent to fluorescence L3. It is noted that a part of passage cover 310 may be made of a material not transparent to fluorescence L3 as long as outcoupling of fluorescence L3 cannot be prevented. An example of the material transparent to fluorescence L3 includes a resin. Passage cover 310 may be connected, without using adhesive layer 350, to metal film 120 (or prism 110) through laser welding, ultrasonic welding, pressure bonding with a clamp member or the like. In this case, the shape of the side surface of passage 320 is defined by passage cover 310.

A pipette tip is inserted into liquid injection port 330. At this point, an opening of liquid injection port 330 (that is, a through hole provided in liquid injection port covering film 331) comes into tight contact with the outer circumference of the pipette tip. Therefore, a liquid can be introduced into passage 320 by injecting the liquid into liquid injection port 330 from the pipette tip, and a liquid held in passage 320 can be removed by sucking the liquid held in liquid injection port 330 into the pipette tip. When injection and suction of a liquid are alternately performed, the liquid can be fed to reciprocate in passage 320.

When a liquid in an amount exceeding the volume of passage 320 is introduced from liquid injection port 330 into passage 320, the liquid flows from passage 320 into reservoir 340. Also when a liquid is fed to reciprocate in passage 320, the liquid flows into reservoir 340. The liquid thus flown into reservoir 340 is stirred within reservoir 340. When the liquid is stirred in reservoir 340, a concentration of a component (such as a BNP or a cleaning component) of the liquid (such as a specimen or a cleaning liquid) passing through passage 320 is made uniform, and hence various reactions can be easily caused in passage 320, or a cleaning effect is improved.

### (Pretreatment of Specimen)

Next, a pretreated specimen is obtained by adding a protease inhibitor to a specimen possibly containing BNP (step S20).

The type of the specimen is not particularly limited. Examples of the specimen include blood, serum, plasma, and a diluted solution thereof. In the detection method for BNP of the present embodiment, BNP is detected by employing SPFS, and hence, whole blood can be used as the specimen.

Addition of protease inhibitor to the specimen is performed by adding, to the specimen, a pretreatment agent containing a protease inhibitor. The pretreatment agent may be a simple protease inhibitor, or may be a composition containing a protease inhibitor. For example, the pretreatment agent is a liquid containing protease. The liquid containing a protease inhibitor can play a role as a diluent for the specimen.

Timing of adding the protease inhibitor (pretreatment agent) to the specimen is not particularly limited as long as the addition is performed at an early stage of the measurement, and is preferably earlier. For example, if the protease inhibitor is held within a blood collecting tube, the protease inhibitor can be added to a specimen at the same time as blood collection. Alternatively, if a specimen is to be diluted before the measurement, the protease inhibitor can be added to the specimen at an early stage of the measurement by using, as a diluent, a liquid containing the protease inhibitor.

The type of the protease inhibitor is not particularly limited as long as it can inhibit decomposition of BNP by erythrocyte-derived protease. Examples of such a protease inhibitor include a serine protease inhibitor and a cysteine protease inhibitor. Examples of the serine protease inhibitor include benzamidine, aprotinin, TLCK (tosyl-L-lysyl-chloromethane hydrochloride), PMFS (phenylmethylsulfonyl fluoride), and AEBSF (aminoethyl benzylsulfonyl fluoride or pefabloc SC). Examples of the cysteine protease inhibitor include aprotinin, PMFS, and AEBSF.

A final concentration (a concentration in the pretreated specimen) of the protease inhibitor may be appropriately set in accordance with the type of the protease inhibitor to be used. For example, if benzamidine is used as the protease inhibitor, a benzamidine concentration in the pretreated specimen is preferably 1 mM or more, more preferably 1.5 mM or more, further preferably 2 mM or more, and particularly preferably 3 mM or more from the viewpoint of prevention of BNP decomposition. The upper limit of the benzamidine concentration in the pretreated specimen is not particularly limited, and is, for example, 10 mM or less, 5 mM or less, or 3.5 mM or less.

Alternatively, if aprotinin is used as the protease inhibitor, an aprotinin concentration in the pretreated specimen is preferably 1 µg/mL or more, more preferably 1.5 µg/mL or more, and further preferably 2 µg/mL or more from the viewpoint of prevention of BNP decomposition. The upper limit of the aprotinin concentration in the pretreated specimen is not particularly limited, and is, for example, 50 µg/mL or less, 10 µg/mL or less, or 5 µg/mL or less.

Alternatively, if TLCK is used as the protease inhibitor, a TLCK concentration in the pretreated specimen is preferably 0.1 mM or more, and more preferably 0.4 mM or more from the viewpoint of prevention of BNP decomposition. The upper limit of the TLCK concentration in the pretreated specimen is not particularly limited, and is, for example, 1 mM or less.

Alternatively, if PMFS is used as the protease inhibitor, a PMFS concentration in the pretreated specimen is preferably 0.1 mM or more, and more preferably 2 mM or more from the viewpoint of prevention of BNP decomposition. The upper limit of the PMFS concentration in the pretreated specimen is not particularly limited, and is, for example, 10 mM or less.

Alternatively, if AEBSF is used as the protease inhibitor, an AEBSF concentration in the pretreated specimen is preferably 0.4 mM or more, and more preferably 2 mM or more from the viewpoint of prevention of BNP decomposition. The upper limit of the AEBSF concentration in the pretreated specimen is not particularly limited, and is, for example, 4 mM or less.

In the step of obtaining the pretreated specimen, a metal protease inhibitor may be added to the specimen in addition to the serine protease inhibitor or the cysteine protease inhibitor. For example, a pretreatment agent containing not only the serine protease inhibitor or the cysteine protease inhibitor but also a metal protease inhibitor may be added. Examples of the metal protease inhibitor include ethylenediaminetetraacetic acid, ethylenediaminetetraacetate, glycol ether diamine tetraacetic acid, and glycol ether diamine tetraacetate. A concentration of the metal protease inhibitor in the pretreated specimen is not particularly limited, and is preferably, for example, 1.5 mg/mL to 1.8 mg/mL.

### (Primary Reaction)

Next, the pretreated specimen is provided onto the metal film of the measurement chip to cause BNP contained in the pretreated specimen to be bound to the binding substance immobilized on the metal film (primary reaction; step S30). A method for providing the pretreated specimen is not particularly limited. For example, a pretreated specimen may be provided onto the metal film using a pipette having a pipette tip attached to a tip thereof. In general, after completing the primary reaction, the surface of the metal film is cleaned with a buffer or the like to remove a component not binding to the binding substance.

In the measurement method of the present embodiment, since a pretreated specimen obtained by adding a prescribed protease inhibitor to a specimen is used, decomposition of BNP otherwise caused by protease leaked from erythrocytes is difficult to occur even if a mechanical stimulus is applied to the pretreated specimen for causing BNP to be bound to a binding substance (for example, even if the pretreated specimen is fed to reciprocate in passage 320 of the measurement chip illustrated in FIG. 3), and hence, reproducibility of measurement results is difficult to degrade.

### (Secondary Reaction)

Next, a labeling reagent is provided onto the metal film of the measurement chip to label, with a fluorescent substance, the BNP having bound to the binding substance (secondary reaction; step S40). A method for providing a labeling reagent is not particularly limited. For example, a labeling reagent may be provided onto the metal film using a pipette having a pipette tip attached to a tip thereof. In general, after completing the secondary reaction, the surface of the metal film is cleaned with a buffer or the like to remove the fluorescent substance not labeling the BNP.

The type of the labeling reagent is not particularly limited as long as the BNP having bound to the binding substance can be labeled with a fluorescent substance. For example, the labeling reagent is a binding substance, labeled with a fluorescent substance, specifically binding to a BNP. The type of the binding substance contained in the labeling reagent is not particularly limited as long as it can specifically bind to a BNP. Examples of the binding substance include an antibody capable of specifically binding to a BNP (an anti-BNP antibody), a nucleic acid capable of specifically binding to a BNP, a lipid capable of specifically binding to a BNP, and a protein, excluding an antibody, capable of specifically binding to a BNP. The binding substance contained in the labeling reagent may be the same type as or different type from the binding substance immobilized on the metal film. When the binding substance is an anti-BNP antibody, the anti-BNP antibody may be a monoclonal antibody, a polyclonal antibody, or a fragment of an antibody. Besides, the number of types of the binding substance contained in the labeling reagent may be one, or may be two or more. For example, the anti-BNP antibody labeled with a fluorescent substance may be one or two or more anti-BNP monoclonal antibodies or anti-BNP polyclonal antibodies. In this case, the anti-BNP monoclonal antibodies and the anti-BNP polyclonal antibodies labeled with a fluorescent substance are preferably different from one or two or more anti-BNP monoclonal antibodies immobilized on the metal film.

The type of the fluorescent substance is not particularly limited as long as it can be used in SPFS. Examples of the fluorescent substance include cyanine-based dyes, Alex Fluor(R) dye of Thermo Scientific, and CF dye of Biotium. Alexa Fluor dye and CF dye have high quantum efficiency for the wavelength of excitation light used in SPFS as compared with other commercially available fluorescent dyes. CF dye is not largely discolored in fluorescence detection, and hence the fluorescence detection can be stably performed. A method for labeling the binding substance with a fluorescent substance is not particularly limited, and can be appropriately selected from known methods. For example, a fluorescent substance may be caused to bind to an amino group or a sulfhydryl group of the binding substance (such as an anti-BNP antibody).

Although a BNP is labeled with the fluorescent substance after causing the BNP to bind to the binding substance immobilized on the metal film in the above description, a BNP may be labeled with the fluorescent substance before causing the BNP to bind to the binding substance immobilized on the metal film. In this case, the specimen or the pretreated specimen may be mixed with the labeling reagent before providing the pretreated specimen onto the metal film. Alternatively, a step of causing the binding substance immobilized on the metal film to bind to BNP and a step of labeling BNP with the fluorescent substance may be simultaneously performed. In this case, the pretreated specimen and the labeling reagent may be simultaneously provided onto the metal film.

### (Fluorescence Measurement)

Next, fluorescence corresponding to the amount of BNP is measured by SPFS (step S50). Specifically, with BNP, labeled with the fluorescent substance, binding to the binding substance immobilized on the metal film, the metal film is irradiated with the excitation light so as to generate SPR on the metal film, and fluorescence thus emitted from the fluorescent substance is measured. In general, a precedently measured optical blank value is subtracted from a measured fluorescent value to calculate a signal value in correlation with the amount of the BNP. If necessary, the signal value may be converted into the amount or concentration of the BNP by using a calibration curve or the like precedently created.

When measurement chip 100 for use in PC-SPFS is used, metal film 120 is irradiated with excitation light L1 through prism 110 as illustrated in FIG. 2A. Thus, SPR is generated in metal film 120, and fluorescent substance 150 present in the vicinity of metal film 120 is excited by the enhanced electric field to emit fluorescence L3. An incident angle of excitation light L1 against metal film 120 is set so as to generate SPR in metal film 120, and is preferably a resonance angle or a reinforcement angle. Here, the term "resonance angle" means an incident angle at which light intensity of reflected light L2 is minimum when the incident angle of excitation light L1 against metal film 120 is scanned. The term "reinforcement angle" means an incident angle at which light intensity of scattered light (plasmon scattered light) of the same wavelength as excitation light L1 emitted upward of metal film 120 (opposite to prism 110) is maximum when the incident angle of excitation light L1 against metal film 120 is scanned.

When measurement chip 200 for use in GC-SPFS is used, metal film 210 (diffraction grating 211) is directly irradiated with excitation light L1 as illustrated in FIG. 2B. Thus, SPR is generated in metal film 210 (diffraction grating 211), and fluorescent substance 150 present in the vicinity of metal film 210 (diffraction grating 211) is excited by the enhanced electric field to emit fluorescence L3. An incident angle of excitation light L1 against metal film 210 is set so as to generate SPR in metal film 210, and is preferably an angle at which intensity of the enhanced electric field formed by SPR is maximum. An optimal incident angle of excitation light L1 is appropriately set in accordance with the pitch of diffraction grating 211, the wavelength of excitation light LI, the type of the metal constituting metal film 210 and the like.

The type of the excitation light is not particularly limited, and is generally laser light. For example, the excitation light is laser light emitted from a laser light source having an output power of 15 to 30 mW. When the output power is 15 mW or more, fluorescence intensity can be increased to appropriately detect the fluorescence. When the output power is 30 mW or less, the binding substance immobilized on the metal film and the like can be prevented from being harmfully affected. The wavelength of the excitation light is appropriately set in accordance with the excitation wavelength of the fluorescent substance to be used.

A detector for the fluorescence is preferably disposed, with respect to the measurement chip, in a direction where the fluorescence intensity is the highest. For example, when measurement chip 100 for use in PC-SPFS is used, the direction where the intensity of fluorescence L3 is the highest is a normal direction of metal film 120 as illustrated in FIG. 2A, and hence, the detector is disposed directly above the measurement chip. On the other hand, when measurement chip 200 for use in GC-SPFS is used, the direction where the intensity of fluorescence L3 is the highest is a direction somewhat inclined against the normal direction of metal film 120 as illustrated in FIG. 2B, and hence, the detector is disposed in a position not directly above the measurement chip. The detector is, for example, a photomultiplier tube (PMT), an avalanche photodiode (APD) or the like.

Through the above-described procedures, the concentration of BNP contained in a specimen can be measured.

### [Measurement Kit for BNP]

A kit for BNP according to the present embodiment is a set of the aforementioned measurement chip, a specimen pretreatment agent containing the protease inhibitor (the serine protease inhibitor or the cysteine protease inhibitor), and the aforementioned labeling reagent. When the measurement chip, the specimen pretreatment agent, and the labeling reagent are thus precedently prepared as a set, a user (such as a health care provider) can more simply perform the measurement method for BNP.

### [Effects]

As described so far, according to the measurement method or measurement kit for BNP of the present embodiment, BNP contained in a specimen can be measured with high sensitivity and higher reproducibility by utilizing SPFS.

### Examples

Now, the present invention will be described in detail with reference to Examples, and it is noted that the present invention is not limited to these Examples.

Experiment: Comparison of Reproducibility of BNP Measured Values

Measurement chip 300 having the structure as illustrated in FIG. 3 was prepared. A mouse anti-BNP monoclonal antibody was immobilized in a specific region (reaction portion) of metal film 120 (metal film) exposed in passage 320.

Blood was collected from a healthy volunteer by using a blood collecting tube holding ethylenediaminetetraacetic acid (EDTA) therein. To the thus obtained blood, five diluents were respectively added to prepare pretreated specimens. A first diluent was an MES buffer containing no protease inhibitor. A second diluent was an MES buffer containing a prescribed amount of aprotinin as a protease inhibitor. A third diluent was an MES buffer containing a prescribed amount of TLCK as a protease inhibitor. A fourth diluent was an MES buffer containing a prescribed amount of benzamidine as a protease inhibitor. A fifth diluent was an MES buffer containing a prescribed amount of EDTA as a protease inhibitor.

In a first pretreated specimen prepared by using the first diluent containing no protease inhibitor, an EDTA concentration was 1.5 mg/mL. In a second pretreated specimen prepared by using the second diluent containing aprotinin, an EDTA concentration was 1.5 mg/mL, and an aprotinin concentration was 1 µg/mL. In a third pretreated specimen prepared by using the third diluent containing TLCK, an EDTA concentration was 1.5 mg/mL, and a TLCK concentration was 0.4 mM. In a fourth pretreated specimen prepared by using the fourth diluent containing benzamidine, an EDTA concentration was 1.5 mg/mL, and a benzamidine concentration was 2 mM In a fifth pretreated specimen prepared by using the fifth diluent containing EDTA, an EDTA concentration was 5.2 mg/mL.

Each pretreated specimen (any one of the first to fifth pretreated specimens) was introduced into passage 320 through liquid injection port 330 with a pipette tip, and fed to reciprocate therein (primary reaction). After removing the pretreated specimen from passage 320 though liquid injection port 330, passage 320 was cleaned once with a cleaning solution. Subsequently, a labeling reagent (a mouse anti-BNP monoclonal antibody labeled, via an amino group, with CF dye (Biotium, Inc.)) was introduced into passage 320 through liquid injection port 330 and fed to reciprocate therein (secondary reaction). After removing the labeling reagent from passage 320 through liquid injection port 330, passage 320 was cleaned three times with a cleaning solution. Subsequently, a measurement liquid was introduced into passage 320 through liquid injection port 330. In this state, a fluorescent value was measured by SPFS. Specifically, metal film 120 was irradiated with excitation light (laser light) from the side of prism 110 with the incident angle of the excitation light against metal film 120 set to a reinforcement angle, and fluorescence emitted at this point was detected. A precedently measured optical blank value was subtracted from the thus obtained fluorescent value to calculate a signal value in correlation with the amount of BNP. The same measurement was performed six times on each of the pretreated specimens.

In order to evaluate reproducibility of the measurement results of each pretreated specimen, a variation coefficient cv (%) of the six measurement results was calculated. It is noted that a lower variation coefficient indicates higher reproducibility, and a higher variation coefficient indicates lower reproducibility. Here, it is evaluated that the reproducibility is sufficiently high if the variation coefficient is 8% or less.

The variation coefficient of the six measurement results of each pretreated specimen is shown in Table 1.

**[Table 1]**

| Pretreated Specimen No. | 1 | 2 | 3 | 4 | 5 |
|---|---|---|---|---|---|
| Protease Inhibitor used in Blood Collecting Tube | EDTA | EDTA | EDTA | EDTA | EDTA |
| Protease Inhibitor used in Diluent | N/A | Aprotinin | TLCK | Benzamidine | EDTA |
| Variation Coefficient (%) | 14.4 | 6.9 | 7.0 | 5.1 | 12.0 |

| | | | | | |
|---|---|---|---|---|---|
| EDTA: metal protease inhibitor Aprotinin: serine protease inhibitor, cysteine protease inhibitor TLCK: serine protease inhibitor Benzamidine: serine protease inhibitor, cysteine protease inhibitor | | | | | |

As is understood from Table 1, the reproducibility of the measurement results was high when the BNP concentration was measured with the serine protease inhibitor or the cysteine protease inhibitor added to the specimen. On the other hand, when the BNP concentration was measured with merely the metal protease inhibitor added to the specimen, the reproducibility of the measurement results was low.

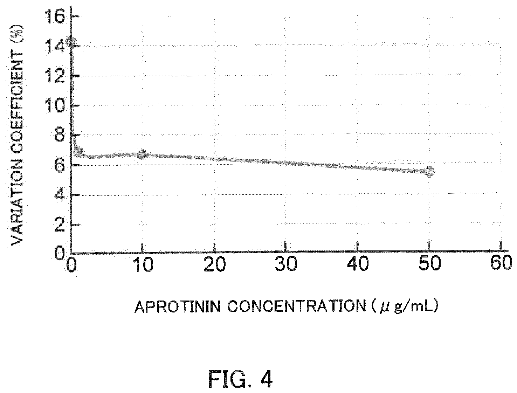

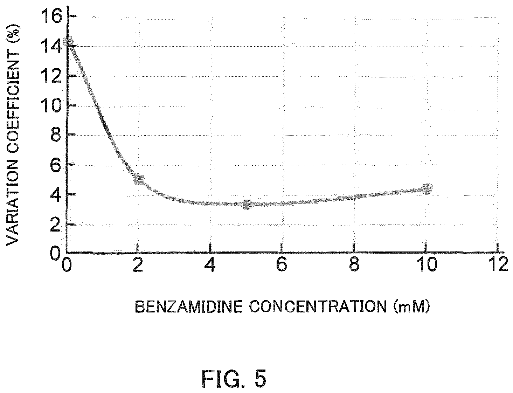

With respect to each of aprotinin and benzamidine, which were found to have an effect of improving the reproducibility of measurement results, the relationship between the inhibitor concentration and the reproducibility of measurement results was checked. Results are respectively illustrated in FIG. 4 and FIG. 5. It is understood from FIG. 4 that the reproducibility of measurement results is good if the aprotinin concentration is 1 µg/mL or more. It is understood from FIG. 5 that the reproducibility of measurement results is good if the benzamidine concentration is 1 mM or more.

The present application is based upon and claims the benefit of priority of Japanese Patent Application No. 2018-147725, filed on August 6, 2018. The entire contents of the specification and drawings thereof are incorporated herein by reference.

### Industrial Applicability

BNP can be measured with high sensitivity and high reproducibility by using a measurement method or measurement kit for BNP according to the present embodiment. Accordingly, the detection method and measurement kit for BNP according to the present invention are useful for, for example, laboratory examinations and the like.

### Reference Signs List

- 100, 200, 300: measurement chip
- 110: prism
- 111: entrance surface
- 112: film surface
- 113: exit surface
- 120: metal film
- 130: anti-brain natriuretic peptide (BNP) antibody
- 140: brain natriuretic peptide (BNP)
- 150: fluorescent substance
- 210: metal film
- 211: diffraction grating
- 310: passage cover
- 320: passage
- 330: liquid injection port
- 331: liquid injection port covering film
- 340: reservoir
- 341: reservoir covering film
- 342: vent
- 350: adhesive layer
- L1: excitation light
- L2: reflected light
- L3: fluorescence

## Claims

1. A measurement method for brain natriuretic peptide, comprising:
preparing a measurement chip including a metal film and a binding substance which is immobilized on the metal film and which specifically binds to brain natriuretic peptide;
obtaining a pretreated specimen by adding, to a specimen containing brain natriuretic peptide, one, two or more protease inhibitors selected from the group consisting of a serine protease inhibitor and a cysteine protease inhibitor;
providing the pretreated specimen onto the metal film to cause the brain natriuretic peptide contained in the pretreated specimen to be bound to the binding substance;
labeling, with a fluorescent substance, the brain natriuretic peptide before or after binding to the binding substance; and
detecting fluorescence emitted from the fluorescent substance when the metal film is irradiated with excitation light in such a manner as to generate surface plasmon resonance in the metal film with the brain natriuretic peptide labeled with the fluorescent substance kept in a state bound to the binding substance.

2. The measurement method for brain natriuretic peptide according to claim 1, wherein the serine protease inhibitor is at least one selected from the group consisting of benzamidine, aprotinin, TLCK, PMFS, and AEBSF.

3. The measurement method for brain natriuretic peptide according to claim 1, wherein the cysteine protease inhibitor is at least one selected from the group consisting of aprotinin, PMFS, and AEBSF.

4. The measurement method for brain natriuretic peptide according to claim 1,
wherein the protease inhibitor includes benzamidine, and
a concentration of the benzamidine in the pretreated specimen is 1 mM or more.

5. The measurement method for brain natriuretic peptide according to claim 1,
wherein the protease inhibitor includes aprotinin, and
a concentration of the aprotinin in the pretreated specimen is 1 µg/mL or more.

6. The measurement method for brain natriuretic peptide according to any one of claims 1 to 5, wherein a metal protease inhibitor is also added to the specimen in the obtaining of the pretreated specimen.

7. The measurement method for brain natriuretic peptide according to claim 6, wherein the metal protease inhibitor is at least one selected from the group consisting of ethylenediaminetetraacetic acid, ethylenediaminetetraacetate, glycol ether diamine tetraacetic acid, and glycol ether diamine tetraacetate.

8. The measurement method for brain natriuretic peptide according to any one of claims 1 to 7,
wherein the metal film is disposed on or above a prism, and
the metal film is irradiated with the excitation light through the prism.

9. The measurement method for brain natriuretic peptide according to any one of claims 1 to 7,
wherein the metal film includes a diffraction grating,
the binding substance is immobilized on the diffraction grating, and
the diffraction grating is irradiated with the excitation light.

10. A measurement kit for brain natriuretic peptide, comprising:
a measurement chip including a metal film, and a binding substance which is immobilized on the metal film and which specifically binds to brain natriuretic peptide;
a pretreatment agent containing one, two or more protease inhibitors selected from the group consisting of a serine protease inhibitor and a cysteine protease inhibitor; and
a labeling reagent for labeling brain natriuretic peptide with a fluorescent substance.

11. The measurement kit for brain natriuretic peptide according to claim 10, wherein the serine protease inhibitor is at least one selected from the group consisting of benzamidine, aprotinin, TLCK, PMFS, and AEBSF.

12. The measurement kit for brain natriuretic peptide according to claim 10, wherein the cysteine protease inhibitor is at least one selected from the group consisting of aprotinin, PMFS, and AEBSF.

13. The measurement kit for brain natriuretic peptide according to claim 10,
wherein the protease inhibitor includes benzamidine, and
the pretreatment agent contains the benzamidine in such a manner as to attain a benzamidine concentration of 1 mM or more after being added to the specimen.

14. The measurement kit for brain natriuretic peptide according to claim 10,
wherein the protease inhibitor includes aprotinin, and
the pretreatment agent contains the aprotinin in such a manner as to attain an aprotinin concentration of 1 µg/mL or more after being added to the specimen.

15. The measurement kit for brain natriuretic peptide according to any one of claims 10 to 14, wherein the labeling reagent is an anti-brain natriuretic peptide antibody labeled with a fluorescent substance.
